# EUROPÄISCHE PATENTSCHRIFT

(11) **EP 3 494 861 B1**
(45) Veröffentlichungstag und Bekanntmachung des Hinweises auf die Patenterteilung: **28.02.2024**
(21) Anmeldenummer: 17205535.2
(22) Anmeldetag: 05.12.2017
(51) Int. Cl.: A61B 1/00, A61B 1/018, A61B 1/005, A61B 17/00

(54) **VORRICHTUNG MIT EINEM ARBEITSKANALFÜHRUNGSELEMENT**
DEVICE WITH A WORKING CHANNEL GUIDE ELEMENT
DISPOSITIF DOTÉ D'UN ÉLÉMENT DE GUIDAGE DU CANAL DE TRAVAIL

(43) Veröffentlichungstag der Anmeldung: 12.06.2019
(73) Patentinhaber: Erbe Elektromedizin GmbH, 72072 Tübingen (DE)
(72) Erfinder: Fischer, Klaus, 72202 Nagold (DE); Herrberg, Charlotte, 72411 Bodelshausen (DE); Stäbler, Thomas, 72070 Tübingen (DE); Brodbeck, Achim, 72555 Metzingen (DE)
(74) Vertreter: Rüger Abel Patentanwälte PartGmbB

(56) Entgegenhaltungen:
- EP-A1- 1 639 936
- WO-A2-2010/118054
- US-A- 6 010 515
- US-A1- 2004 039 250
- US-A1- 2012 165 604
- US-A1- 2013 041 214
- US-A1- 2015 335 388
- US-B1- 6 878 106

## Beschreibung

Die Erfindung betrifft eine Vorrichtung mit einem Endoskop und einem mittels des Endoskops geführten Instrument.

Aus dem Stand der Technik sind Vorrichtungen umfassend ein Endoskop bekannt, durch dessen Arbeitskanal sich ein Instrument über das distale Ende des Endoskops hinaus erstreckt, wobei das Instrument in dem Arbeitskanal längsverschiebbar geführt ist. DE 103 34 100 A1 beispielsweise beschreibt ein Endoskop mit einem abwinkelbaren Abschnitt eines Arbeitskanals für einen solchen Zweck.

Aus DE 10 2006 054 218 A1 ist ein Endoskop bekannt, durch dessen Arbeitskanal sich ein Führungsdraht erstreckt, wobei am distalen Ende im Führungskanal ein am Führungsdraht angeschlossener Okluder angeordnet ist, der zum Verschließen einer Incision mittels des Führungsdrahts aus dem Führungskanal ausschiebbar ist.

Bei anderen Vorrichtungen ist das Instrument au-ßen an dem Endoskop geführt.

In der in EP 3 141 203 A1 offenbarten Vorrichtung, beispielsweise, erstreckt sich ein Ablationsinstrument entlang des Schaftes eines Instruments durch ein Lumen einer Schlauchhülle, die ein anderes Lumen bereitstellt, durch das sich das Endoskop erstreckt. Das Ablationsinstrument erstreckt sich außerdem durch eine Fassung, die am distalen Ende des Schaftes des Endoskops befestigt ist. Das Ablationsinstrument ist in dem Lumen der Schlauchhülle und in der Fassung beweglich entlang des Schaftes des Endoskops geführt.

Aus DE 10 2010 020 220 A1 ist eine Führungshülse bekannt, die an einer Längsseite des Schaftes eines Endoskops befestigt werden kann, um durch die Führungshülse endoskopische Instrumente neben dem Endoskop zu führen.

DE 20 2009 009 342 U1 beschreibt ein Endoskop mit einem flexiblen Endoskopschaft, an dessen Längsseite ein aufblasbarer Schlauch angeordnet ist.

US 6 010 515 A zeigt ein Endoskop, in dessen Arbeitskanal der Schaft eines Fadenführungselements verschiebbar angeordnet ist. Das Fadenführungselement kann dazu verwendet werden, einen Knoten zu einer Stelle zu bewegen, an welcher der Knoten angebracht werden soll.

US 2012 / 0 165 604 A1 zeigt eine Vorrichtung mit einem optischen Endoskop und einem Instrument mit einem länglichen, röhrenförmigen Gehäuse sowie einer Nadel. Das Instrument ist mit einem Befestigungselement außen an dem Endoskop befestigt. Das Endoskop erstreckt sich in einer Hülle.

US 6 878 106 B1 zeigt ein Endoskop mit einem Arbeitskanal, durch welchen sich ein Instrument zum Greifen einer Schlinge erstreckt. Die Schlinge schaut aus dem Ende des Endoskops heraus. Durch die Schlinge ist ein zusätzliches Instrument geschoben.

Es ist Aufgabe der vorliegenden Erfindung, eine verbesserte Vorrichtung mit einem Endoskop und einem mittels des Endoskops geführten Instruments anzugeben.

Diese Aufgabe wird mit einer erfindungsgemäßen Vorrichtung gemäß Anspruch 1 gelöst:
Die erfindungsgemäße Vorrichtung weist ein Endoskop mit einem Schaft auf, wobei der Schaft wenigstens einen Arbeitskanal beinhaltet. Der Arbeitskanal mündet an einer Seite (Austrittsseite) des Schaftes, vorzugsweise an der Stirnseite des Endoskops, an dessen distalem Ende. Der Schaft ist vorzugsweise flexibel. Die Vorrichtung weist wenigstens ein chirurgisches Instrument auf, das neben dem Schaft verlaufend angeordnet ist. Die Vorrichtung ist derart eingerichtet, dass ein distaler Endabschnitt des Instruments, der den Arbeitsabschnitt des Instruments enthält, entlang des Schaftes über die Seite des Schaftes, an der der Arbeitskanal mündet, hinaus bewegbar ist, um den Arbeitsabschnitt des Instruments in einen Bereich gegenüber (vor) der Seite des Schaftes zu bewegen, um mit dem Arbeitsabschnitt in diesem Bereich bei dem endoskopischem Einsatz der Vorrichtung zu arbeiten. Der Bereich ist vorzugsweise mit Hilfe eines Mittels des Endoskops zur Bildübertragung einsehbar. Das Instrument steht mit einem Führungselement in Eingriff, um den Arbeitsabschnitt des Instruments mittels des Führungselements bei der Bewegung des Endabschnitts über die Seite des Schaftes hinaus in den Bereich zu führen. Zur Führung des Abschnitts des Instruments erstreckt sich das Führungselement während der Bewegung des distalen Endabschnitts über die Seite des Schaftes hinaus durch den Arbeitskanal. Das Instrument ist derart mit dem Führungselement bewegungsgekoppelt, so dass ein Vorschieben des Führungselements in dem Arbeitskanal in Längserstreckungsrichtung des Arbeitskanals in Richtung zu der Austrittsseite in eine Bewegung des Arbeitsabschnitts des Instruments von der Austrittsseite weg umgesetzt wird. Vorzugsweise ist das Führungselement in dem Arbeitskanal längsbeweglich geführt, um mit dem Schaft eine Teleskopführung für den Arbeitsabschnitt des entlang der Längsseite des Schaftes angeordneten Instruments zu bilden.

Dass das Instrument mittels des Führungselements geführt wird, umfasst, dass das Führungselement derart mit dem Instrument bewegungsgekoppelt ist, dass das Instrument bei einer Bewegung des in dem Arbeitskanal längsbeweglich geführten Führungselements mit dem Führungselement mitbewegt wird und/oder dass das Führungselement bei einer Bewegung des Instruments entlang des Schaftes mit dem Instrument mitbewegt wird, wobei das in dem Arbeitskanal längsbeweglich geführte Führungselement dem Instrument die Führung des Führungselements in dem Arbeitskanal vermittelt. Alternativ oder zusätzlich umfasst, dass das Instrument mittels des Führungselements erfindungsgemäß geführt wird, dass an dem Führungselement eine Führung zur Führung des Instruments außerhalb des Arbeitskanals angeordnet ist, wobei die Führung vorzugsweise gegenüber der Seite angeordnet ist, an der der Arbeitskanal aus dem Schaft austritt, und wobei das Instrument mit der Führung in Eingriff steht.

Die erfindungsgemäße Vorrichtung erlaubt eine im Vergleich zu bekannten Vorrichtungen stabilere Führung des außen an dem Endoskop angeordneten Instruments und damit beispielsweise eine zügige Positionierung und/oder Ausrichtung des Arbeitsabschnitts des Instruments in dem Bereich, in dem mit dem Instrument gearbeitet werden soll, beispielsweise relativ zu dem mit dem Instrument zu behandelnden und/oder untersuchenden Gewebe.

Die erfindungsgemäße Vorrichtung wird vorzugsweise durch wenigstens eines der nachfolgend beschriebenen Merkmale weitergebildet:

Die Vorrichtung ist vorzugsweise derart eingerichtet, dass das Führungselement entlang des Arbeitskanals in distale Richtung vorgeschoben und/oder in proximale Richtung zurückgezogen werden kann und/oder dass das Führungselement in dem Arbeitskanal gedreht werden kann, wobei die Bewegung des Führungselements durch eine Bewegungskopplung des Führungselements mit dem Instrument auf das Instrument übertragen wird. Dabei kann das Führungselement zur Bewegung des Instruments mittels des Führungselements vorzugsweise von außerhalb des Körpers des Patienten bedient werden, während das distale Ende der Vorrichtung mit dem Arbeitsabschnitt innerhalb des Körpers angeordnet ist.

Vorzugsweise wird das Vorschieben in eine Bewegung des Arbeitsabschnitts in Längserstreckungsrichtung des Arbeitskanals weg von der Stirnseite umgesetzt. Alternativ oder zusätzlich ist das Instrument vorzugsweise derart mit dem Führungselement gekoppelt, dass ein Zurückziehen des Führungselements in dem Arbeitskanal in Längserstreckungsrichtung des Arbeitskanals in Richtung zu dem proximalen Ende der Vorrichtung in eine Bewegung des Arbeitsabschnitts des Instruments zurück näher an den Schaft umgesetzt wird. Der Arbeitsabschnitt ist durch Krafteinleitung in das Führungselement vorzugsweise in distale Richtung vorschiebbar und/oder vorziehbar. Zusätzlich oder alternativ ist der Arbeitsabschnitt vorzugsweise durch Krafteinleitung in das Führungselements in proximale Richtung zurückziehbar und/oder zurückschiebbar.

Die Vorrichtung ist dazu eingerichtet, dass das Führungselement in dem Arbeitskanal um die Längsachse des Führungselements drehbar ist, wobei die Drehbewegung auf Grund der Kopplung des Führungselements mit dem Instrument auf den Arbeitsabschnitt übertragen wird, um den Arbeitsabschnitt um eine Achse zu bewegen und/oder zur schwenken. Der Arbeitsabschnitt des Instruments ist über das Führungselement zu einer Drehung und Verschwenkung um die Längsachse des Führungselements antreibbar. Zusätzlich ist das Instrument derart mit dem Führungselement bewegungsgekoppelt, dass der Arbeitsabschnitt des Instruments geschwenkt wird, wenn ein aus dem Schaft herausstehender, von der Austrittsseite abstehender Abschnitt des Führungselements relativ zu dem Schaft gegenüber der Stirnseite um eine Achse quer zur Längserstreckungsrichtung des Führungselements geschwenkt wird.

Bei dem Führungselement kann es sich um einen flexiblen Führungsstab handeln. Durch das Führungselement kann sich längs des Führungselements ein Hohlraum erstrecken. Das Führungselement kann entsprechend beispielsweise ein flexibles Rohr und/oder einen Schlauch umfassen. Das Führungselement kann beispielsweise einen Draht oder ein Bündel von Drähten umfassen. Der Draht oder die Drähte können sich entlang des Arbeitskanals erstrecken. Alternativ oder zusätzlich kann das Führungselement beispielsweise eine Drahtwendel umfassen. Das Führungselement kann beispielsweise aus Kunststoff und/oder Metall bestehen.

Bei zumindest einem der mittels des Führungselements geführten Instrumente kann es sich beispielsweise um ein Probenentnahmeinstrument und/oder ein Applikationsinstrument zur Applikation von Flüssigkeit, Gas und/oder Feststoffen an das Gewebe und/oder in das Gewebe, ein Absauginstrument und/oder ein chirurgisches Schneid- und/oder Koagulationsinstrument handeln. Bei wenigstens einem der mittels des Führungselements geführten, seitlich neben dem Schaft verlaufend angeordneten Instrumente kann es sich alternativ oder zusätzlich um ein Messinstrument zum Regeln, Steuern, Messen oder Erfassen von Gewebeparametern handeln. Alternativ oder zusätzlich kann ein mittels des Führungselements geführtes Instrument beispielsweise der Energieübertragung an das distale Ende der Vorrichtung dienen.

Bei zumindest einem der mittels des Führungselements geführten Instrumente kann es sich beispielsweise um eine Koagulationssonde, eine chirurgische Flüssigkeitsstrahlsonde zur Anhebung (Elevation) von Gewebe durch Einbringen von Flüssigkeit unter das Gewebe, eine Kryosonde, beispielsweise eine Kryosonde zum Festfrieren und Entnehmen einer Gewebeprobe, eine Probenexzisionszange, eine Injektionskanüle, einen Fremdkörper- und/oder Steinfänger, eine Bürste und/oder einen Absaugkatheter handeln.

Die Funktion des Führungselements kann auf das Bereitstellen einer Führung für das eine oder die mehreren Instrumente, die mit dem Führungselement in Eingriff stehen, und/oder auf die Übertragung von Bewegungen auf das eine oder die mehreren Instrumente beschränkt sein, die mit dem Führungselement in Eingriff stehen. Alternativ ist das Führungselement selbst ein medizinisches Instrument. Insbesondere kann das Führungselement einen führungselementeigenen Arbeitsabschnitt aufweisen. Durch das Führungselement kann sich beispielsweise ein Kanal zur Leitung von Flüssigkeit, Gas und/oder Feststoff erstrecken. Der sich durch das Führungselement erstreckende Kanal kann zur Absaugung von Flüssigkeit, Gas und/oder Feststoff von dem distalen Ende des Kanals und/oder zum Transport von Flüssigkeit, Gas und/oder Feststoff an das distale Ende und aus dem distalen Ende heraus bestimmt sein.

Das Instrument weist vorzugsweise wenigstens ein Funktionselement auf, das sich neben dem Schaft des Endoskops bis zu dem Arbeitsabschnitt des Endoskops erstreckt. Das Funktionselement ist vorzugsweise zur Führung und/oder Bewegung des Instruments, insbesondere dessen Arbeitsabschnitts, und/oder zur Betätigung des Instruments und/oder zur Übertragung von Medien, wie etwa Flüssigkeit, Gas, Feststoff und/oder elektrischem Strom zu dem Arbeitsabschnitt und/oder von dem Arbeitsabschnitt weg eingerichtet. Das Funktionselement ist vorzugsweise derart eingerichtet, dass der Benutzer der Vorrichtung das Instrument mittels des Funktionselement bewegen und/oder führen und/oder bedienen und/oder versorgen kann, wobei die Handhabung und/oder die Versorgung des Instruments mittels des Funktionselement vorzugsweise von außerhalb des Körpers des Patienten erfolgen kann, während der Arbeitsabschnitt des Instruments in dem Körper des Patienten angeordnet ist.

Vorzugsweise ist, zusätzlich zu der Führung mittels des Führungselements, an der Längsseite des Schaftes eine Führung angeordnet, die zum Führen des Instruments entlang der Längsseite des Schaftes eingerichtet ist. Die Führung steht mit dem Instrument, vorzugsweise mit dem sich bis zu dem Arbeitsabschnitt des Instruments entlang des Schaftes erstreckenden Funktionselement des Instruments, in Eingriff. Bevorzugt ist das Funktionselement an dem Schaft mittels wenigstens eines zwischen der Längsseite des Schaftes und dem Instrument wirksam angeordneten Führungshalters geführt. Ein Führungshalter kann beispielsweise an dem Schaft befestigt sein. Alternativ ist ein Führungshalter beispielsweise an dem Funktionselement befestigt. Bei einem Führungshalter kann es sich beispielsweise um einen Führungsring handeln, der an dem Schaft oder dem Funktionselement befestigt ist und durch den sich der Schaft und/oder das Funktionselement erstrecken.

Das Führungselement weist vorzugsweise wenigstens einen Eingriffsabschnitt zur Kopplung des Führungselements mit einem Instrument auf. Vorzugsweise ist das Führungselement zur Kopplung an zwei oder mehr Instrumente eingerichtet. Das Führungselement weist vorzugsweise je einen Eingriffsabschnitt für zwei oder mehrere Instrumente auf.

Alternativ oder zusätzlich kann an dem Führungselement ein Eingriffselement befestigt sein, wobei das Führungselement mittels des Eingriffselements mit wenigstens einem Instrument koppelbar ist.

Vorzugsweise steht das Instrument derart mit dem Eingriffselement und/oder dem Eingriffsabschnitt in Eingriff, dass das Instrument relativ zu dem Eingriffselement und/oder dem Eingriffsabschnitt verschiebbar in dem Eingriffselement und/oder dem Eingriffsabschnitt geführt ist. Das Führungselement stellt damit vorzugsweise mittels des Eingriffselements und/oder mittels des Eingriffsabschnitts eine Führung für das Instrument außerhalb des Schaftes gegenüber der Austrittsseite bereit, aus der das Führungselement aus dem Schaft austritt.

Bevorzugt weist das Eingriffselement und/oder der Eingriffsabschnitt eine zu der äußeren Form des distalen Endes des Schaftes passende Form und/oder eine zu einem an dem distalen Ende des Schaftes angeordneten Halteelement passende Form auf, so dass das Eingriffselement und/oder der Eingriffsabschnitt eine festgelegte Orientierung und/oder eine festgelegte Position relativ zu dem distalen Ende des Schaftes und/oder relativ zu dem Halteelement einnimmt, wenn die passende Form mit der äußeren Form in Eingriff steht. Beispielsweise ist an dem distalen Ende des Schaftes eine Fassung für das Eingriffselement und/oder den Eingriffsabschnitt angeordnet und/oder ausgebildet, wobei das Eingriffselement und/oder der Eingriffsabschnitt axial in die Fassung beweglich sind und wobei die Drehbeweglichkeit des Eingriffselements und/oder des Eingriffsabschnitts in der Fassung eingeschränkt ist. Die Vorrichtung ist vorzugsweise derart eingerichtet, dass die äußere Form und die passende Form beim Zurückbewegen des Eingriffsabschnitts und/oder des Eingriffselements in Richtung zu dem Schaft automatisch in Eingriff gelangen.

Der Eingriff zwischen dem Führungselement und dem Instrument ist vorzugsweise zerstörungsfrei lösbar. Besonders bevorzugt ist die Vorrichtung derart eingerichtet, dass der Eingriff zwischen dem Führungselement und dem Instrument von außerhalb des Körpers des Patienten zerstörungsfrei lösbar ist und/oder dass ein zerstörungsfrei lösbarer Eingriff zwischen dem Führungselement und dem Instrument von außerhalb des Körpers des Patienten herstellbar ist, wenn das distale Ende der Vorrichtung mit dem Arbeitsabschnitt in dem Körper des Patienten angeordnet ist.

An dem distalen Ende des Schaftes ist vorzugsweise ein Führungshalter befestigt, der eine Führung der sich entlang des Schaftes erstreckenden Instrumente entlang des Schaftes bereitstellt. Dazu weist der Führungshalter vorzugsweise Aufnahmen auf, in denen je ein Instrument verschiebbar, vorzugsweise entlang der Längserstreckung des distalen Endes des Schaftes verschiebbar, geführt ist. Die Vorrichtung ist vorzugsweise dazu eingerichtet, dass mit dem Führungselement jeweils ein oder mehrere Instrumente in Eingriff gebracht werden können, um diese mittels des Führungselements zu bewegen, während wenigstens ein anderes in seiner Aufnahme angeordnetes Instrument außer Eingriff mit dem Führungselement steht.

Bevorzugt ist das Führungselement zur Aufnahme wenigstens zweier Instrumente, die neben dem Schaft verlaufend angeordnet sind, derart eingerichtet, dass der Arbeitsabschnitt wenigstens eines der Instrumente relativ zu dem Arbeitsabschnitt eines anderen der Instrumente, mittels des Führungselements geführt, verschiebbar ist. Alternativ oder zusätzlich ist das Führungselement vorzugsweise zur Aufnahme wenigstens zweier Instrumente, die neben dem Schaft verlaufend angeordnet sind, derart eingerichtet, dass der Arbeitsabschnitt wenigstens eines der Instrumente relativ zu dem Arbeitsabschnitt des anderen der Instrumente geführt mittels des Führungselements drehbar und/oder schwenkbar ist. Bevorzugt ist die Vorrichtung zur Bedienung des Führungselements zum Verschieben, Drehen und/oder Schwenken eines der Instrumente relativ zu dem anderen Instrument von außerhalb des Körpers des Patienten eingerichtet, wenn die Arbeitsabschnitte der Instrumente in dem Körper des Patienten angeordnet sind.

Erfindungsgemäß wird auch ein Führungselement angegeben, das zur Verwendung in einer erfindungsgemäßen Vorrichtung, beispielsweise wie hierin beschrieben, eingerichtet ist.

Weitere vorteilhafte Merkmale der erfindungsgemäßen Vorrichtung ergeben sich aus der nachfolgenden Beschreibung, den Figuren sowie den Unteransprüchen.

### FIGURENBESCHREIBUNG

Es zeigen schematisch:
Figuren 1a bis 1d - ausschnittsweise perspektivische Ansichten von Ausführungsbeispielen der erfindungsgemäßen Vorrichtung,
Figur 1e - ein weiteres Ausführungsbeispiel der erfindungsgemäßen Vorrichtung in einer ausschnittsweise perspektivischen Darstellung,
Figur 2a - eine ausschnittsweise perspektivische Darstellung eines weiteren Ausführungsbeispiels der erfindungsgemäßen Vorrichtung mit einem an dem Endoskop befestigten Führungshalter mit Aufnahmen für Instrumente,
Figur 2b - eine ausschnittsweise perspektivische Darstellung eines Ausführungsbeispiels einer Anordnung eines Endoskops und eines Führungselements einer beispielhaften erfindungsgemäßen Vorrichtung, wobei mit dem Führungselement wenigstens zwei Instrumente vermittelt über das Führungselement relativ zueinander bewegt werden können,
Figur 2c - ein Ausführungsbeispiel der erfindungsgemäßen Vorrichtung in einer ausschnittsweise perspektivischen Darstellung mit einem Führungselement und einem relativ dazu verschiebbaren Instrument,
Figuren 3ai bis 3d - perspektivische Darstellungen verschiedener Varianten der Ankopplung des Führungselements an das Eingriffselement bzw. das Instrument,
Figur 4 - ein beispielhafter Handgriff für eine erfindungsgemäße Vorrichtung.

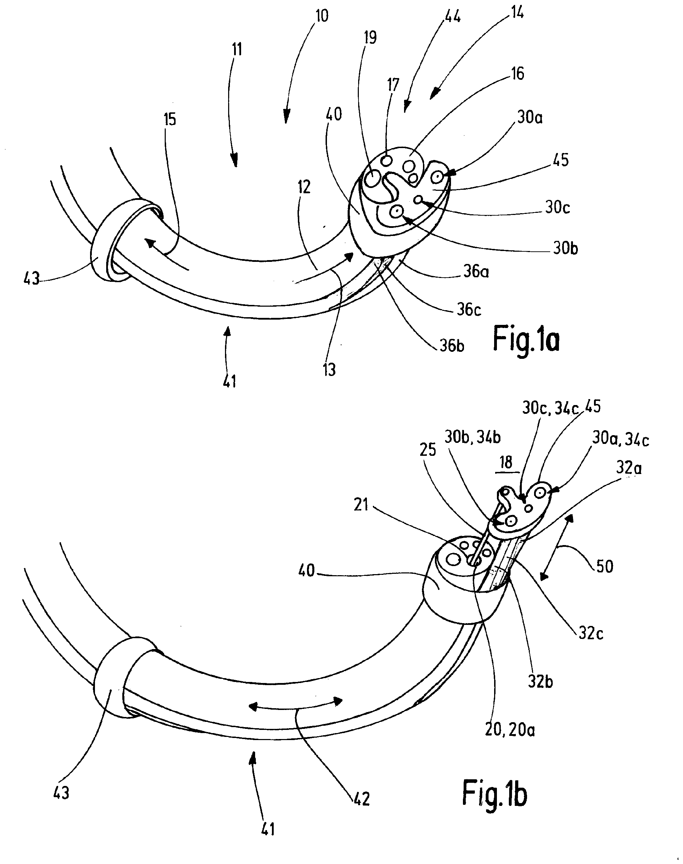

Die Figuren 1a, 1b zeigen eine beispielhafte Ausführungsform der erfindungsgemäßen Vorrichtung 10 in einer ausschnittsweise perspektivischen Ansicht. Die Vorrichtung 10 weist ein Endoskop 11 mit einem flexiblen Schaft 12 auf. Der Schaft 12 legt eine distale Richtung 13 (Richtungssinn) fest, entlang derer sich der Schaft 12 von seinem proximalen Ende (nicht dargestellt) bis zu seinem distalen Ende 14 erstreckt. Umgekehrt legt der Schaft 12 eine proximale Richtung 15 von seinem distalen Ende 14 entlang des Schaftes 12 bis zu seinem proximalen Ende fest. An der Stirnseite 16 des Schaftes 12 an dessen distalem Ende 14 ist eine Beleuchtungseinheit 17 zur Beleuchtung eines Bereichs 18 gegenüber der Stirnseite 16 des Schaftes 12 angeordnet. Durch den Schaft 12 erstreckt sich ein Mittel zur Übertragung eines Bildes bis zu einem Eingang 19 des Mittels in der Stirnseite 16, an dem beispielsweise ein zu dem Mittel zur Bildübertragung gehörendes Objektiv angeordnet sein kann. Das Mittel ist zur Übertragung eines Bildes von dem zu untersuchenden und/oder behandelnden Bereich 18 gegenüber der Stirnseite 16 des Schaftes 12 eingerichtet, um die Untersuchung und/oder Behandlung in dem Bereich 18 vis-a-vis der Stirnseite 16 mittels des Endoskops 11 von außerhalb des Körpers des Patienten visuell überwachen zu können. Durch den Schaft 12 können sich bis zu der Stirnseite 16 noch weitere Leitungen zur Übertragung in proximale Richtung und/oder distale Richtung, beispielsweise von Signalen, elektrischer Leistung, Gasen, Flüssigkeiten oder Feststoffen, erstrecken. Durch den Schaft 12 erstreckt sich jedenfalls wenigstens ein Arbeitskanal 20 bis zu einer Öffnung 21 in der Stirnseite 16 des Schaftes 12. Durch einen solchen Arbeitskanal 20 kann bei einer bekannten Vorrichtung 10 beispielsweise ein Instrument, wie etwa ein Werkzeug, z.B. ein Greifer, aus der Stirnseite 16 des Schaftes 12 heraus geschoben werden, um mit diesem in dem Bereich 18 mit Hilfe des Mittels zur Bildübertragung unter visueller Kontrolle eines Operateurs Untersuchungen und/oder Behandlungen in dem Bereich 18 durchzuführen.

In der erfindungsgemäßen Vorrichtung 10 erstreckt sich (siehe insbesondere Figur 1b) durch den Arbeitskanal 20 ein längliches Führungselement 25, das in dem Arbeitskanal 20 in Längsrichtung 26 des Arbeitskanals 20 bewegbar geführt ist. Das Führungselement 25 tritt durch die Öffnung 21 in der Stirnseite (Austrittsseite), aus dem Arbeitskanal 20 aus. Das Führungselement 25 erstreckt sich in proximale Richtung 15 vorzugsweise bis zu einem proximalen Ende der Vorrichtung 10 (in Figur 1a, 1b nicht dargestellt) von wo aus das Führungselement 25 von einem Operateur betätigbar, insbesondere bewegbar ist.

Die erfindungsgemäße Vorrichtung 10 umfasst wenigstens ein Instrument 30, das sich neben dem Schaft 12 entlang des Schaftes 12 erstreckt. In dem dargestellten Ausführungsbeispiel gehören zu der erfindungsgemäßen Vorrichtung 10 ein erstes 30a und ein zweites Instrument 30b, bei denen es sich jeweils um eine Koagulationssonde mit jeweils wenigstens einer Elektrode zum Zünden eines Plasmas handeln kann, und ein drittes Instrument 30c, bei dem es sich beispielsweise um eine Wasserstrahlsonde handeln kann. Die Instrumente 30a, 30b, 30c weisen jeweils einen distalen Endabschnitt 32a-c mit einem Arbeitsabschnitt 34a-c an den distalen Enden der Instrumente 30a, 30b, 30c auf.

Zu dem Arbeitsabschnitt 34a-c erstreckt sich je Instrument 30a, 30b, 30c wenigstens ein Funktionselement 36a-c längs des Schaftes 12. Die seitlich neben dem Schaft 12 verlaufenden Funktionselemente 36a-c können zur Führung und/oder Bewegung des jeweiligen Instruments 30a-c bzw. des Arbeitsabschnitts 34a-c und/oder zur Betätigung des Instruments 30a-c und/oder zur Übertragung von Medien zu dem Arbeitsabschnitt 34a-c des Instruments 30a-c und/oder von dem Arbeitsabschnitt 34a-c am distalen Ende des Instruments 30a-c weg eingerichtet sein. Der Benutzer der Vorrichtung 10 kann das Instrument 30a-30c über das Funktionselement 36a-c beispielsweise bewegen, führen, bedienen und/oder versorgen. Wenn es sich bei dem ersten und dem zweiten Instrument 30a, 30b um eine APC-Sonde handelt, kann das erste Instrument 30a und das zweite Instrument 30b als Funktionselement 36a, 36b beispielsweise jeweils einen Schlauch aufweisen, durch den sich jeweils eine elektrische Leitung erstreckt, die mit der Elektrode verbunden ist, wobei durch den Schlauch Argongas zu dem Arbeitsabschnitt 34a, 34b der APC-Sonde mit deren Elektrode geleitet werden kann, um vor dem distalen Ende der APC-Sonde ein Argonplasma zu zünden. Wenn es sich bei dem dritten Instrument 30c, wie in dem dargestellten Ausführungsbeispiel, um eine Flüssigkeitsstrahlsonde handelt, weist diese als Funktionselement 36c beispielsweise einen Schlauch auf, der an einer Mündung der Flüssigkeitsstrahlsonde in dem Arbeitsabschnitt 34c der Sonde an deren distalem Ende angeschlossen ist und der dem Zuführen einer Flüssigkeit zu der Mündung dient.

Die Funktionselemente 36a-c der Instrumente 30a-c erstrecken sich in dem dargestellten Ausführungsbeispiel durch eine an dem distalen Ende 14 des Schaftes 12 befestigte Führungsmanschette 40, die einen an der Längsseite 41 des Schaftes 12 angebrachten ersten Führungshalter 40 der Vorrichtung 10 zur Führung der Instrumente 30a, 30b, 30c außen an dem Schaft 12 in Längserstreckungsrichtung 42 des Schaftes 12 bildet. In proximale Richtung 15 von dem distalen Ende 14 und dem ersten Führungshalter 40 beabstandet erstrecken sich die Funktionselemente 36a-c durch einen an der Längsseite 41 des Schaftes 12 angeordneten zweiten Führungshalter 43, der dazu eingerichtet ist, die Funktionselemente 36a-c an der Längsseite 41 des Schaftes 12 zu führen. Bei dem zweiten Führungshalter 43 kann es sich beispielsweise um einen Ring handeln, der den Schaft 12 und/oder die Funktionselemente 36a-c umschließt. Der zweite Führungshalter 43 kann gegen axiale Bewegung entlang des Schaftes 12 an dem Schaft 12 fixiert sein oder an dem Funktionselement 36a-c gegen eine axiale Bewegung des zweiten Führungshalters 43 relativ zu den Funktionselementen 36a-c fixiert sein. Zusätzlich zu den beiden dargestellten beispielhaften Führungshaltern 40, 43 zur Führung des oder der seitlich neben dem Schaft 12 verlaufend angeordneten Instrumente 30a, 30b, 30c kann außen an dem Schaft 12 wenigstens ein weiterer Führungshalter (nicht dargestellt) zur Führung des oder der seitlich entlang des Schaftes 12 verlaufend angeordneten Instrumente 30a, 30b, 30c angebracht sein. Es sind auch Ausführungsformen mit nur einem zwischen der Längsseite 41 des Schaftes 12 und dem oder den Instrumenten 30a, 30b, 30c wirksamen Führungshalter zur Führung der Instrumente 30a, 30b, 30c an dem Schaft entlang der Längsseite 41 des Schaftes 12 möglich.

Der an dem distalen Ende 14 des Schaftes 12 angeordnete erste Führungshalter 40 kann ein Gleitelement bilden, über welches die Vorrichtung 10 auf einem Gewebeabschnitt aufliegt, um die Einhaltung eines definierten Abstands des Arbeitsabschnitts wenigstens eines Instruments 30a, 30b, 30c zum Gewebe zu erleichtern.

Zusätzlich oder alternativ zu den ersten und/oder zweiten Führungshaltern 40, 43 kann die Vorrichtung 10 einen Folienschlauch (nicht dargestellt) aufweisen, wobei sich der Schaft 12 und die Funktionselemente 36a-c durch den Folienschlauch erstrecken, wobei der Folienschlauch vorzugsweise ein Lumen für den Schaft 12 und ein davon gesondertes Lumen für die Funktionselemente 36a-c bereitstellt, wobei die Funktionselemente 36a-c in dem Lumen für die Funktionselemente 36a-c an dem Schaft 12 des Endoskops 11 geführt werden. Der Folienschlauch kann zusätzlich der Rauchabsaugung dienen.

Die Instrumente 30a-c sind am distalen Ende 44 der Vorrichtung 10 mit dem Führungselement 25, das sich durch den Arbeitskanal 20 erstreckt, mit einem Eingriffsabschnitt des Führungselements 25 und/oder mit einem mit dem Führungselement 25 gekoppelten Eingriffselement 45 derart gekoppelt, dass die Instrumente 30a, 30b, 30c bei einer Bewegung des Arbeitsabschnitts 34a-c weg von der Stirnseite 16 des Schaftes 12 in den Bereich gegenüber (vis-a-vis) der Stirnseite 16 und/oder zurück zu der Stirnseite 16 aus dem Bereich 18 heraus von dem während der Bewegung in dem Arbeitskanal 20 geführten Führungselement 25 geführt werden. Dazu ist der Abschnitt des Führungselements 25, der aus der Stirnseite 16 distal herausschaut und der sich bis in den Arbeitskanal 20 erstreckt, biegesteif oder, vorzugsweise, biegeelastisch, um die Instrumente 30a, 30b, 30c zu führen.

Die Vorrichtung 10 ist derart ausgebildet, die Gegenzwangskräfte auf Grund der Führung der Instrumente 30a, 30b, 30c über das Führungselement 25, durch die den Kanal 20 begrenzende, den Kanal 20 umschließende Wandfläche in den Schaft 12 abzuleiten. Das Führungselement 25 kann sich demnach innerhalb des Arbeitskanals 20 an dem Schaft 12 abstützen und vermittelt dadurch dem neben dem Schaft 12 angeordneten Instrument 30a, 30b, 30c die Führung des Führungselements 25 in dem Arbeitskanal 20. Das Instrument wird also gegenüber der Austrittsseite 16 außerhalb des Arbeitskanals 20 vermittelt über das Führungselement 25 mit dem Arbeitskanal 20 geführt. Der Schaft 12 und das Führungselement 25 bilden demnach eine Teleskopführung für die Arbeitsabschnitte 34a-c der entlang der Längsseite 41 des Schaftes 12 verlaufend angeordneten Instrumente 30a, 30b, 30c. Da bei der erfindungsgemäßen Vorrichtung 10 der Arbeitskanal 20 vermittelt über das Führungselement 25 zur Führung der Arbeitsabschnitte 34a-c gegenüber der Austrittsseite 16 genutzt wird, können die Arbeitsabschnitt 34a-c vor der Austrittsseite 16 stabiler und damit sicherer und zügiger als bei bekannten Vorrichtungen geführt werden.

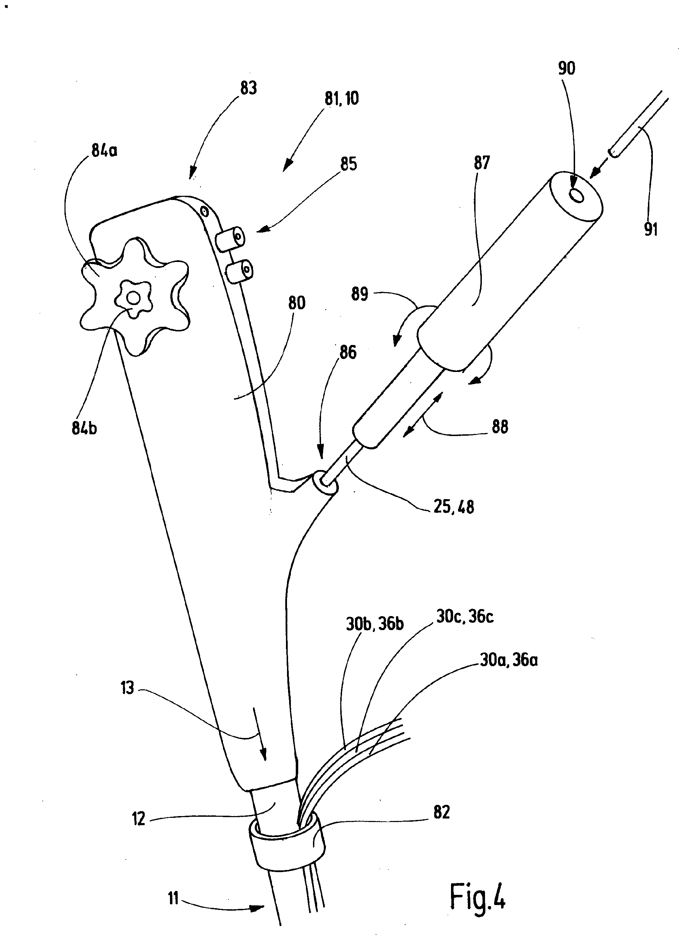

Die erfindungsgemäße Vorrichtung 10 ist vorzugsweise derart eingerichtet, dass der Benutzer die Kraft in Bewegungsrichtung zur Bewegung der Arbeitsabschnitte 34a-c der Instrumente 30a, 30b, 30c in den Bereich 18 gegenüber der Stirnseite 16 und/oder aus dem Bereich 18 heraus auf die Arbeitsabschnitte 34a-c über das Führungselement 25 übertragen kann. Vorzugsweise ist die Vorrichtung 10 derart eingerichtet, dass der Benutzer das Führungselement 25 am Handgriff (in Figur 1a, 1b nicht dargestellt, s. z.B. Figur 4) der Vorrichtung 10 bedienen, z.B. in distaler Richtung 13 vorschieben, kann, um eine Kraft zur Bewegung der Arbeitsabschnitte 34a-c in den Bereich 18 und/oder aus dem Bereich 18 heraus entlang des Führungselements 25 auf die Instrumente 30a, 30b, 30c zu übertragen. Die Vorrichtung 10 ist dadurch derart eingerichtet, dass der Benutzer das Führungselement 25 von außerhalb des Körpers des Patienten zur Krafteinleitung zum Schieben und/oder Ziehen der Instrumente 30a, 30b, 30c aus dem Bereich 18 zurück und/oder in den Bereich 18 vor über das Führungselement 25 bedienen kann. Beim Schieben und/oder Ziehen der Instrumente 30a, 30b, 30c über das Führungselement 25 kann es erforderlich sein, die Funktionselemente 36a-c (Leitungen) der Instrumente 30a, 30b, 30c von außerhalb des Körpers des Patienten aus nachzuschieben oder zurückzuziehen.

Um eine unmittelbare Übertragung einer Schubbewegung des betätigten Abschnitts 48 des Führungselements 25, z.B. am Handgriff, auf eines oder mehrere der Instrumente 30a, 30b, 30c zu erreichen, ist das Führungselement 25 vorzugsweise drucksteif und mit dem Arbeitsabschnitt 34a-c des Instruments 30a, 30b, 30c vorzugsweise drucksteif gekoppelt. Zudem ist das ein Schubglied bildende Führungselement 25 in dem Arbeitskanal 20 vorzugsweise biegesteif geführt.

Um eine unmittelbare Übertragung einer Zugbewegung des Führungselements 25 am Handgriff auf das Instrument 30a, 30b, 30c zu erreichen, ist das Führungselement 25 vorzugsweise zugsteif und vorzugsweise zugsteif mit dem Arbeitsabschnitt 34a, 34b, 34c des Instruments 30a, 30b, 30c gekoppelt.

Der Endabschnitt 20a des Arbeitskanals 20 an der Öffnung 21 in der Stirnseite 16 gibt mit seiner Längserstreckungsrichtung eine Führungsrichtung 50 für das Führungselement 25 und damit die Arbeitsabschnitte 34a, 34b, 34c der Instrumente 30a, 30b, 30c vor, entlang derer das Führungselement 25 und darüber auch die Arbeitsabschnitte 34a, 34b, 34c der Instrumente 30a, 30b, 30c von der Stirnseite 16 weg in den Bereich 18 und zurück geführt werden. Die distalen Endabschnitte 32a-c der Instrumente 30a, 30b, 30c sind, wie in Figur 1b gezeigt, in Führungsrichtung 50 entlang der Längserstreckungsrichtung des distalen Endabschnitts des Arbeitskanals 20 durch Krafteinleitung in das Führungselement 25 von dem distalen Ende 14 des Schaftes 12 weg bewegbar (vorschiebbar und/oder vorziehbar) und/oder, in umgekehrte Richtung, zu dem distalen Ende 14 des Schaftes 12 hin zurückbewegbar (zurückschiebbar und/oder zurückziehbar) .

Alternativ oder zusätzlich zur Krafteinleitung in das Führungselement 25 zur Bewegung der neben der Längsseite 41 des Schaftes 12 verlaufenden Instrumente 30a, 30b, 30c kann die Vorrichtung 10 derart eingerichtet sein, dass eine Kraft in Führungsrichtung 50 zur Bewegung der Instrumente entlang der Längserstreckungsrichtung des Endabschnitts des Arbeitskanals 20 auf die Arbeitsabschnitte 34a-c der Instrumente 30a, 30b, 30c ganz oder teilweise über wenigstens eines der Funktionselemente 36a-c übertragen wird. In solchen Fällen kann der Benutzer beispielsweise die Funktionselemente 36a-c entlang des Schaftes 12 in distale Richtung 13 vorschieben, um die Abschnitte 32a-c der Instrumente 30a, 30b, 30c mit deren Arbeitsabschnitten 34a, 34b, 34c über die Stirnseite 16 hinaus in Richtung zu dem Bereich 18 zu bewegen, in dem mit den Arbeitsabschnitten 34a-c gearbeitet werden soll.

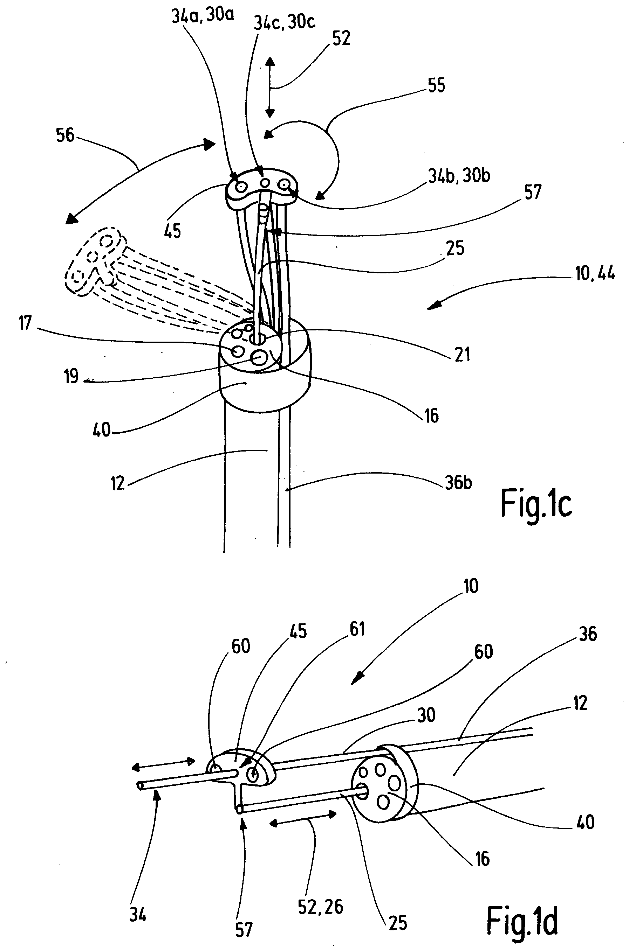

Figur 1c zeigt ein Ausführungsbeispiel der erfindungsgemäßen Vorrichtung 10, die beispielsgemäß derart eingerichtet ist, dass die Arbeitsabschnitte 34a-c um eine entlang der Längserstreckungrichtung 52 des Führungselements 25 durch das Führungselement 25 verlaufende Achse relativ zu dem Schaft 12 rotiert und/oder geschwenkt werden können, wie es durch den Doppelpfeil 55 angedeutet ist. Dies schließt auch eine Rotation und/oder ein Schwenken mit einem Rotationswinkel und/oder Schwenkwinkel zwischen einer Anfangs- und einer Endstellung ein, der kleiner als 360° ist. Die Vorrichtung 10 ist vorzugsweise dazu eingerichtet, dass das Drehmoment für die Rotation und/oder das Schwenken auf die Arbeitsabschnitte 34a, 34b, 34c über das Führungselement 25 übertragen werden kann. Bevorzugt kann das Führungselement 25 zur Rotation der Arbeitsabschnitte 34a, 34b, 34c und/oder zum Verschwenken derselben von außerhalb des Körpers des Patienten bedient werden, wenn das distale Ende 44 der Vorrichtung 10 in einem Lumen des Körpers angeordnet ist. Das Führungselement 25 ist dazu vorzugsweise in zumindest einem Drehsinn, beschränkt auf einen Drehsinn oder in beiden Drehsinnen torsionssteif, um eine unmittelbare Übertragung einer Drehbewegung des Führungselements 25 auf die Instrumente 30a, 30b, 30c zu erreichen.

Wie in Figur 1c anhand des Doppelpfeils 56 und der gestrichelten Darstellung des aus der Öffnung 21 herausschauenden Abschnitts des Führungselements 25, des Eingriffselements 45, und der Instrumente 30a, 30b, 30c in einer verschwenkten Stellung erkennbar, sind die gegenüber der Stirnseite 16 angeordneten Arbeitsabschnitte 34a, 34b, 34c bei der beispielhaften Ausführungsform relativ zu dem distalen Ende 14 des Schaftes um eine quer, beispielsweise senkrecht, zu dem Führungselement 25 verlaufende Achse schwenkbar, um die Orientierung der gegenüber der Stirnseite 16 angeordneten Arbeitsabschnitte 34a, 34b, 34c zu verändern. Die Vorrichtung 10 ist vorzugsweise derart eingerichtet, dass das Drehmoment für das Schwenken auf die Instrumente 30a, 30b, 30c über das Führungselement 25 übertragen werden kann. Beispielsweise kann das Führungselement 25 einen oder mehrere mit dem distalen Ende 57 des Führungselements 25 gekoppelte Drähte (nicht dargestellt) umfassen, die sich bis zu einem Handhabungsabschnitt der Vorrichtung beispielsweise am Handgriff der Vorrichtung erstrecken, so dass der Benutzer der Vorrichtung 10 die Drähte von außerhalb des Körpers des Patienten bedienen kann, wenn das distale Ende 44 der Vorrichtung 10 innerhalb des Lumens eines Körpers des Patienten angeordnet ist. Das Führungselement 25 kann beispielsweise aus einem hohlen biegeelastischen Rohr gebildet sein, wobei sich die Drähte von dem proximalen Ende der Vorrichtung 10 bis zu dem distalen Ende 57 des Führungselements 25 durch das Rohr erstrecken können.

Figur 1d zeigt ein weiteres Ausführungsbeispiel der erfindungsgemäßen Vorrichtung 10 mit einem mit dem Führungselement 25 gekoppelten Eingriffselement 45, das wenigstens eine Aufnahme 60 für ein Instrument aufweisen kann, um das Instrument derart über das Eingriffselement 45 mit dem Führungselement 25 zu koppeln, dass eine in das Führungselement 25 eingeleitete Kraft in Längserstreckungsrichtung des Führungselements 25 auf das Instrument (der Übersichtlichkeit halber hier nicht dargestellt) übertragen wird. Das Eingriffselement 45 weist eine Aufnahme 61 für ein weiteres Instrument 30 auf, die dazu eingerichtet ist, das weitere Instrument 30, vorzugsweise längs der Längserstreckungsrichtung 52 des aus der Öffnung distal herausschauenden Abschnitts des Führungselements 25, in dem Eingriffselement 45 gegenüber der Stirnseite 16 relativ zu dem Führungselement 25 verschiebbar zu führen. Alternativ zu einem mit dem Führungselement 25 gekoppelten Eingriffselement 45 kann auch ein Eingriffsabschnitt des Führungselements 25 eine derartige Aufnahme für ein weiteres Instrument 30 bereitstellen. Das Führungselement 25 bildet einen über die Stirnseite 16 vorstehenden, in dem Arbeitskanal 20 gehaltenen Führungsausleger für das relativ zu dem Führungselement 25 verschiebbare weitere Instrument 30 und stellt mit dem Eingriffselement 45 oder dem Eingriffsabschnitt eine Führung für das weitere Instrument 30 bereit, die gegenüber (vor) der Stirnseite 16 positioniert ist. Das Führungselement 25 ist in dem Arbeitskanal 20 entlang der Längserstreckungsrichtung des Arbeitskanals 20 verschiebbar geführt, so dass der Abstand des Eingriffselements 45 oder des Eingriffsabschnitts und damit der Abstand der Führung für das weitere Instrument 30 von der Stirnseite 16 veränderbar ist. Die Vorrichtung 10 kann derart eingerichtet sein, dass die Kraft zur Verschiebung des Arbeitsabschnitts 34 des weiteren Instruments 30 relativ zu dem Führungselement 25 auf den Arbeitsabschnitt 34 über das Funktionselement 36 des Instruments 30 eingeleitet werden kann. Das Funktionselement 36 kann dazu neben der Längsseite des Schaftes durch einen Führungshalter 40 geführt sein. Bei dem weiteren Instrument 30 kann es sich beispielsweise um eine Flüssigkeitsstrahlsonde handeln. Eine solche kann dazu eingerichtet sein, Flüssigkeit unter eine Gewebeschicht einzutragen, um diese abzuheben. Dabei wird das distale Ende der Flüssigkeitsstrahlsonde auf das Gewebe aufgesetzt. Mit der beispielhaften Ausführungsform der erfindungsgemäßen Vorrichtung kann das distale Ende der Flüssigkeitsstrahlsonde beabstandet von dem Eingriffselement 45 und der Stirnseite 16 auf das Gewebe aufgesetzt werden. Mit dem über das distale Ende des Führungselements 25 hinaus weg von dem distalen Ende 57 des Führungselements 25 verschiebbaren distalen Arbeitsabschnitt 34 des weiteren Instruments 30 kann auch in enge Lumen des Körpers des Patienten vorgedrungen werden.

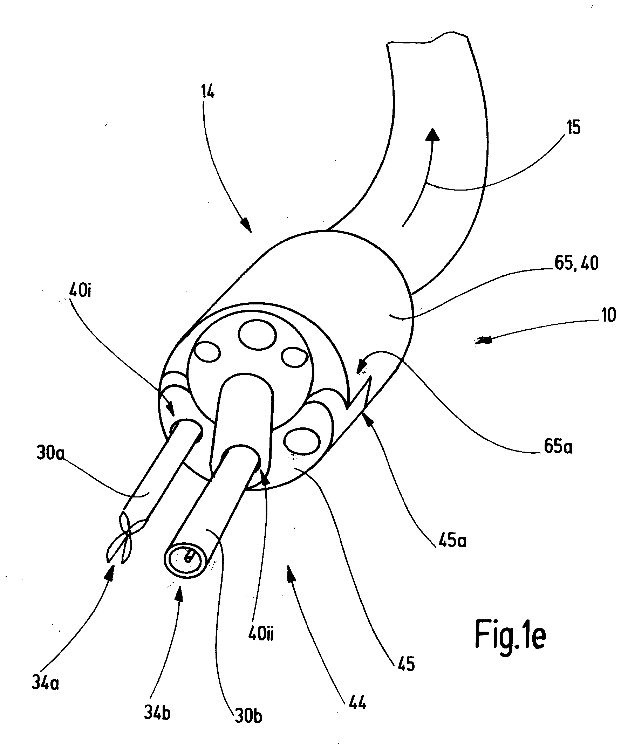

Figur 1e ist eine ausschnittsweise, perspektivische Darstellung eines weiteren Ausführungsbeispiels der erfindungsgemäßen Vorrichtung 10 in einer Ansicht auf das distale Ende 44 der Vorrichtung 10. An dem distalen Ende 14 des Schaftes 12 ist ein Halteelement 65 mit einem Formschlussabschnitt 65a befestigt. Der Formschlussabschnitt 65a weist eine zu einem Formschlussabschnitt 45a des Eingriffselements 45 komplementäre Form auf. In Figur 1e ist das Eingriffselement 45 in einer zurückgezogenen Stellung am Schaft 12 angeordnet, so dass der Formschlussabschnitt 45a des Eingriffselements 45 mit dem Formschlussabschnitt 65a des Halteelement 65 in Eingriff steht. Der Formschluss bewirkt, dass das Eingriffselement 45 um den Schaft 12 drehfest an dem Halteelement 65 angeordnet ist und dass das Eingriffselement 45 nicht weiter in proximale Richtung 15 relativ zu dem distalen Ende 14 des Schaftes 12 zurückbewegt werden kann. Das Halteelement 65 legt damit eine bestimmte Orientierung und Position des Eingriffselements 45 am Schaft 12 in der zurückgezogenen Stellung fest. Das Haltelement 65 kann gleichzeitig eine Führung für die Instrumente 30a, 30b am Schaft bilden. Beispielsweise kann es sich bei dem Haltelement 65 um einen ersten Führungshalter 40 mit wenigstens zwei gesonderten Aufnahmen 40i, 40ii für je ein Instrument 30a, 30b handeln. Das Instrument 30a kann beispielsweise einen Arbeitsabschnitt 34a mit einem Greifer aufweisen. Das Instrument 30b kann beispielsweise eine APC-Sonde einen Arbeitsabschnitt 34b mit einer Elektrode sein, um ein Argonplasma zu erzeugen.

An dem Halteelement 65 und an dem Eingriffselement 45 sind vorzugsweise Führungs- und Gegenführungsflächen angeordnet (nicht dargestellt), die beim Zurückziehen des Eingriffselements 45 an den Schaft 12 miteinander derart in Eingriff gelangen, dass der Formschlussabschnitt 45a des Eingriffselements 45 automatisch in Formschluss mit dem Formschlussabschnitt 65a des an dem distalen Ende 14 des Schaftes 12 befestigten Halteelements 65 gelangt. Auf diese Weise kann die Position und die Orientierung des Eingriffselements 45 an dem Schaft 12 automatisch mittels des Halteelements 65 eingestellt werden, wenn das Eingriffselement 45 aus einer vorgeschobenen Stellung gegenüber der Stirnseite 16 zurückbewegt wird.

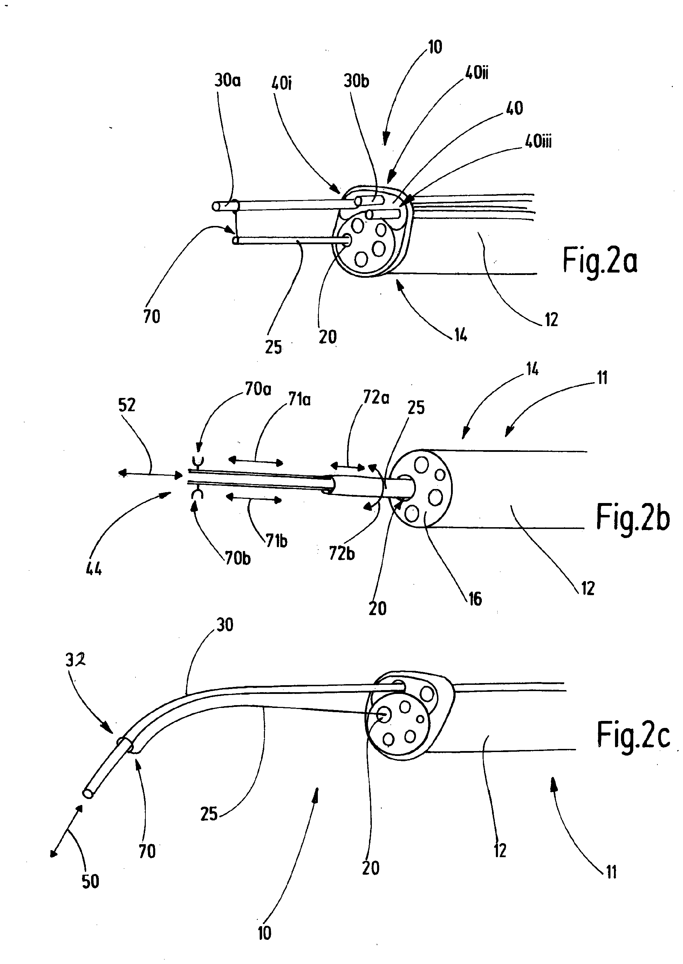

Figur 2a zeigt perspektivisch, ausschnittsweise eine beispielhafte Ausführungsform der erfindungsgemäßen Vorrichtung 10 mit einem an dem distalen Ende 14 des Schaftes 12 befestigten Führungshalter 40, der Aufnahmen 40i, 40ii, 40iii aufweist, in denen je ein Instrument 30a, 30b, 30c geführt angeordnet ist. Die Instrumente 30a, 30b, 30c sind in den Aufnahmen 40i, 40ii, 40iii vorzugsweise entlang der Längserstreckungsrichtung des distalen Endabschnitts des Arbeitskanals 20 verschiebbar geführt. Das sich durch den Arbeitskanal 20 erstreckende Führungselement 25 weist einen Eingriffsabschnitt 70 auf. Die Vorrichtung 10 ist dazu eingerichtet, dass das Führungselement 25 über dessen Eingriffsabschnitt 70 wahlweise mit wenigstens zwei der Instrumente 30a, 30b, 30c gekoppelt werden kann, um das jeweils mit dem Führungselement 25 gekoppelte Instrument 30a in den Bereich 18 gegenüber der Stirnseite 16 zu führen, in dem mit dem Instrument 30a gearbeitet werden soll, und/oder das Instrument 30a aus dem Bereich 18 zurück näher an die Stirnseite 16 herauszuführen. Die Vorrichtung 10 ist vorzugsweise derart eingerichtet, dass das Führungselement 25 von dem Instrument 30a entkoppelt werden kann, um das Führungselement 25 mit einem der anderen Instrumente 30b, 30c zu koppeln, um dieses Instrument 30b, 30c in den Bereich 18 gegenüber der Stirnseite 16 zu führen, in dem mit dem Instrument 30b, 30c gearbeitet werden soll, und/oder dieses andere Instrument 30b, 30c aus dem Bereich 18 gegenüber der Stirnseite 16 herauszuführen. Die Vorrichtung 10 ist vorzugsweise dazu eingerichtet, dass die Kraft längs der Längserstreckungsrichtung 52 des Führungselements 25 zur Bewegung des Instruments 30a, 30b, 30c in den Bereich 18 und/oder die Kraft längs der Längserstreckungsrichtung 52 des Führungselements 25 zur Bewegung aus dem Bereich 18 heraus auf das Instrument 30a, 30b, 30c über das Führungselement 25 übertragen wird. Die Vorrichtung 10 ist vorzugsweise derart eingerichtet, dass die Kopplung eines Instruments 30a, 30b, 30c mit dem Führungselement 25 und/oder die Entkopplung eines Instruments 30a, 30b, 30c von dem Führungselement 25 vorzugsweise von außerhalb des Körpers des Patienten bedient werden kann, während das distale Ende 44 der Vorrichtung 10 mit den Arbeitsabschnitten 34a-c der Instrumente 30a, 30b, 30c innerhalb des Körpers des Patienten angeordnet ist.

Figur 2b zeigt eine perspektivische, ausschnittsweise Darstellung einer beispielhaften Ausführungsform eines Endoskops 11 der erfindungsgemäßen Vorrichtung 10 mit einem in dessen Arbeitskanal 20 längsverschieblich geführten Führungselement 25. Das Führungselement 25 weist zwei Eingriffsabschnitte 70a, 70b auf, die jeweils zumindest an ein Instrument ankoppeln können, dass sich entlang des Schaftes 12 erstreckt, um dieses vis-a-vis der Stirnseite 16 des Schaftes 12 zu führen. Die Instrumente sind der Übersichtlichkeit halber in Figur 2b nicht dargestellt. Das Führungselement 25 ist dazu eingerichtet, dass der Arbeitsabschnitt 34a, 34b jedes der mit den Eingriffsabschnitten 70a, 70b gekoppelten Instrumente, wie mit den Doppelpfeilen 71a, 71b angedeutet, relativ zu dem Arbeitsabschnitt des anderen Instruments in Längserstreckungsrichtung 52 des Führungselements 25 verschoben werden können. Außerdem können die Arbeitsabschnitte der mit den Eingriffsabschnitten 70a, 70b gekoppelten Instrumente durch Drehen und/oder Schwenken der Eingriffsabschnitte 70a, 70b um eine längs der Längserstreckungsrichtung 52 des Führungselements 25 verlaufende Achse relativ zueinander verschwenkt werden und/oder um die längs der Längserstreckungsrichtung 52 verlaufende Achse rotiert werden. Zudem ist die Vorrichtung 10 dazu eingerichtet, dass das Führungselement 25 als ganzes entlang seiner Längsachse in dem Arbeitskanal 20 geführt vorgeschoben und zurückgezogen und um seine Längsachse gedreht werden kann wie mit den Doppelpfeilen 72a,b angedeutet, um die Arbeitsabschnitte beider Instrumente entsprechend bewegen zu können. Die Kraft zur Bewegung der Arbeitsabschnitte relativ zueinander und/oder die Kraft zur Bewegung des Führungselements 25 als ganzes kann vorzugsweise von außerhalb des Körpers des Patienten in das Führungselement 25 eingeleitet werden, während das distale Ende 14 des Schaftes 12 in dem Körper des Patienten angeordnet ist. Damit kann die Stellung des Arbeitsabschnitts eines Instruments relativ zu dem anderen Arbeitsabschnitt von außerhalb des Körpers des Patienten geändert werden, während das distale Ende 44 der Vorrichtung 10 mit den Arbeitsabschnitten innerhalb des Körpers des Patienten angeordnet ist.

Figur 2c zeigt ein Ausführungsbeispiel der erfindungsgemäßen Vorrichtung 10 mit einem Führungselement 25, dass sich durch den Arbeitskanal 20 des Schaftes 12 des Endoskops 11 erstreckt, wobei das Führungselement 25 beispielsweise einen gebogenen Endabschnitt aufweisen kann, an dem der Eingriffsabschnitt 70 des Führungselements 25 angeordnet ist. In dem Eingriffsabschnitt 70 ist der distale Endabschnitt 32 des Instruments 30 längs einer Führungsrichtung 50 beweglich geführt, die der Eingriffsabschnitt 70 vorgibt. Die Führungsrichtung 50 ist von der Längserstreckungsrichtung des distalen Endabschnitts des Arbeitskanals 20 abgewinkelt, um den Arbeitsabschnitt 34 des Instruments 30 in einen Bereich weg von der gedachten parallelen Verlängerung der Mittelachse des distalen Endabschnitts des Arbeitskanals 20 weg zu führen.

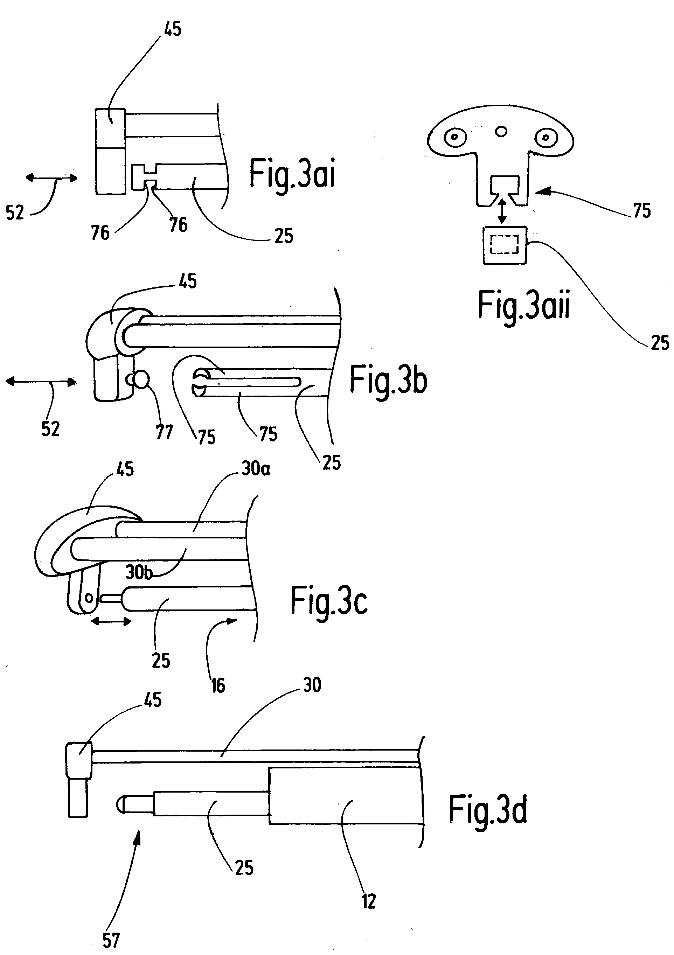

Die Figuren 3ai bis 3d zeigen verschiedene Ausführungsbeispiele der Befestigung des Führungselements 25 an dem Eingriffselement 45. Die Befestigungen sind zur Kraftübertragung von dem Führungselement 25 auf die Instrumente 30a, 30b geeignet, um die Arbeitsabschnitte 34a, 34b der an dem Eingriffselement 45 befestigten Instrumente 30a, 30b durch Krafteinleitung in das Führungselement 25 weg von der Austrittsseite 16 zu bewegen und/oder die Arbeitsabschnitte 34a, 34b der Instrumente 30a, 30b zurück an die Austrittsseite 16 zubewegen. Die Figuren 3ai und 3aii zeigen eine Schnappverbindung zur Herstellung eines Formschlusses zwischen dem Eingriffselement 45 und dem Führungselement 25 quer zur Längserstreckungsrichtung 52 des Führungselements 25. Der Formschluss zwischen Eingriffselement 45 und Führungselement 25 in Längserstreckungsrichtung 52 des Führungselements wird dadurch ausgebildet, dass der Abschnitt des einen Verbindungspartners (hier des Eingriffselements 45) mit dem wenigstens einen Schnappelement 75, den anderen Verbindungspartner, hier das Führungselement 25, bei hergestellter Schnappverbindung zwischen gegenüberliegenden Formschlussflächen 76 umgreift, die an dem anderen Verbindungspartner ausgebildet sind, wobei die Formschlussflächen 76 eine Bewegung des einen Verbindungspartners relativ zu dem anderen Verbindungspartner in Richtung 52 der Längserstreckung des Führungselements 25 verhindern. In dem Ausführungsbeispiel gemäß Figur 3b wird eine Formschlussverbindung quer zur Längserstreckungsrichtung 52 des Führungselements 25 und in Längserstreckungsrichtung 52 des Führungselements 25 durch einen kugelförmigen Körper 77 an dem Eingriffselement 45 hergestellt, die bei hergestellter Verbindung von zwei gegenüberliegenden Schnappzungen 75 umschlossen wird. Figur 3c zeigt ein Ausführungsbeispiel bei dem der Formschluss in Bewegungsrichtung nur bei einem Schieben des Eingriffselements 45 mittels des Führungselements 25 von der Austrittsseite 16 weg gegeben ist. Wenn jedoch das Führungselement 25 zurückgezogen wird, kann dadurch das Führungselement 25 aus der Aufnahme in dem Eingriffselement 45 herausgezogen werden, um die Instrumente 30a, 30b von dem Führungselement 25 zu trennen. Bei der Ausführungsform gemäß Figur 3d wird in Bewegungsrichtung des Führungselements 25 ein Kraftschluss dadurch hergestellt, dass ein distaler Endabschnitt 57 des Führungselements 25, der ein Übermaß bezüglich eines inneren Maßes einer Ausnehmung in dem Eingriffselement 45 aufweist, in der Ausnehmung angeordnet wird. Unabhängig von der konkreten Ausführungsform ist die Verbindung zwischen dem Instrument 30a, 30b und dem Führungselement 25 vorzugsweise am distalen Ende der Vorrichtung lösbar, um einen Instrumentenwechsel zu ermöglichen, ohne das Instrument 30a, 30b von der Vorrichtung 10 entfernen zu müssen.

Figur 4 zeigt einen beispielhaften Handgriff 80 einer erfindungsgemäßen Vorrichtung 10, der an dem proximalen Ende 81 der Vorrichtung 10 angeordnet ist. Der Handgriff 80 ist an den Schaft 12 des Endoskops 11 angeschlossen. Um den Schaft 12 ist ein Führungshalter 82 benachbart zu dem Handgriff 80 angeordnet ist, durch den sich Funktionselemente 36a, 36b, 36c von Instrumenten 30a, 30b, 30c bis zu dem Arbeitsabschnitten 34a, 34b, 34c der Instrumente 30a, 30b, 30c an dem distalen Ende 44 der Vorrichtung 10 erstrecken. Der Handgriff 80 weist einen Bedienteil 83 zur Bedienung des Endoskops 11 auf. Beispielsweise kann der Bedienteil 83 Abwinkelungsräder 84a, 84b zur gezielten Krümmung des distalen Endes 14 des Endoskops 11 und Bedienelemente 85, beispielsweise für die Luft- und/oder Wasserzufuhr an das distale Ende 14 des Schaftes 12, aufweisen. An dem Handgriff 80 ist ein Eingang 86 in den Handgriff 80 angeordnet, der mit dem sich durch den Schaft 12 erstreckenden Arbeitskanal 20 verbunden ist und durch den sich das Führungselement 25 in den Arbeitskanal 20 des Endoskops 11 erstreckt. An dem Führungselement 25 ist ein an dem Handgriff 80 angeordneter Führungselementgriff 87 befestigt, über den das Führungselement 25 weiter in Richtung 13 zu dem distalen Ende 14 des Schaftes 12 vorgeschoben und/oder das Führungselement 25 etwas aus dem Eingang 86 zurückgezogen werden kann, wie durch den Doppelpfeil 88 angedeutet ist. Zudem kann der Führungselementgriff 87 gedreht werden (Doppelpfeil 89), um das Führungselement 25 um die Längsachse des Führungselements 25 zu drehen. Damit kann der Eingriffsabschnitt 70, 70a, 70b des Führungselements 25 und/oder das mit dem Führungselement 25 gekoppelte Eingriffselement 45 mittels des Führungselementgriffs 87 gegenüber der distalen Stirnseite 16 des Schaftes 12 relativ zu der Stirnseite 16 von außerhalb des Körpers des Patienten bewegt, positioniert und/oder ausgerichtet werden, wenn der Arbeitsabschnitt 34a-c des mit dem Führungselement 25 über dessen Eingriffsabschnitt 70, 70a, 70b und/oder über das Eingriffselement 45 gekoppelten Instruments 30a, 30b innerhalb des Körpers des Patienten angeordnet ist. Der Führungselementgriff 87 selbst kann optional direkt oder indirekt über den Eingang 86 zu dem Arbeitskanal 20, beispielsweise mittels eines Luer-Lock-Anschlusses, an dem Handgriff 80 befestigt sein, wobei sich der befestigte Führungselementgriff 87 vorzugsweise entlang des Doppelpfeils 88 zur Vor- und Zurückbewegung des mit dem Führungselement 25 gekoppelten Instruments 30a, 30b, 30c bewegen lässt und/oder sich, wie durch den Doppelpfeil 89 angedeutet, um eine Längsachse drehen lässt, um das mit dem Führungselement 25 gekoppelte Instrument 30a, 30b, 30c zu drehen bzw. zu schwenken. Mit der Befestigung des Führungselementgriffs 87 kann der Arbeitskanal 20 abgedichtet sein. Bei einer indirekten Befestigung dient ein Adapter der Befestigung am Endoskop 11 und der Abdichtung des Arbeitskanals 20.

Das Führungselement 25 kann zum Führen von Flüssigkeit, Gas, Feststoff und/oder eines Instruments innerhalb des Führungselements 25 hohl sein. An dem Führungselementgriff 87 kann beispielsweise eine Öffnung 90 angeordnet sein, durch die Flüssigkeit, Gas und/oder Feststoff von dem distalen Ende 44 der Vorrichtung 10 durch das Führungselement 25 bis zu der Öffnung 90 gesaugt werden kann, und/oder durch die Flüssigkeit, Gas und/oder Feststoff in den Bereich 18 vor der Stirnseite 16 des Endoskops 11 gebracht werden kann. Alternativ oder zusätzlich kann durch die Öffnung 90 ein Instrument 91 durch das Führungselement 25 in den Bereich 18 vor der Stirnfläche 16 geschoben werden und/oder aus dem Bereich 18 zurückgezogen werden. Bei dem Instrument kann es sich beispielsweise um eine flexible Wasserstrahl- bzw. Flüssigkeitsstrahlsonde handeln.

Das Führungselement 25 kann elektrisch leitfähig sein, wobei außerhalb des Körpers des Patienten eine elektrische Leistungsquelle an das Führungselement 25 angeschlossen sein kann, die zur Versorgung wenigstens eines der Instrumente 30a-c eingerichtet sein kann, die neben dem Schaft 12 des Endoskops 11 geführt sind.

Mit der erfindungsgemäßen Vorrichtung 10 kann beispielsweise wie folgt gearbeitet werden: Zu Beginn können die Instrumente 30a, 30b, 30c in der Stellung wie in Figur 1a gezeigt angeordnet sein, in der das Führungselement 25 in einer maximal in den Arbeitskanal 20 eingezogenen Stellung ist. Der Benutzer führt das distale Ende 14 des Endoskops 11 sodann in den Körper des Patienten ein. Dabei kann der Benutzer die Krümmung des distalen Endes 14 des Schaftes 12 beispielsweise mittels der Abwinkelungsräder 84a, 84b am Handgriff 80 anpassen. Am Zielort angekommen, kann der Benutzer die Position und/oder die Orientierung der Arbeitsabschnitte 34a-c der Instrumente 30a-30c relativ zu dem distalen Ende 14 des Schaftes 12 durch Krafteinleitung in das Führungselement 25 verändern. Beispielsweise kann der Benutzer die Arbeitsabschnitte 34a-34c der Instrumente 30a-30c in eine Richtung quer zur Stirnseite 16 von der Stirnseite 16 weg, beispielsweise senkrecht von der Stirnseite weg, durch Bedienung des Führungselements 25 von außerhalb des Körpers des Patienten bewegen, in dem der Benutzer das Führungselement 25 durch den Arbeitskanal 20 weiter in Richtung zu dem distalen Ende 14 des Schaftes 12 schiebt. Dadurch können die Arbeitsabschnitte 34a-c beispielsweise in einer Stellung relativ zu dem distalen Ende 14 des Schaftes 12 wie in Figur 1b dargestellt, angeordnet werden. Vor oder während der Behandlung oder Untersuchung in dem Bereich 18, in dem die Arbeitsabschnitte 34a-c angeordnet sind, kann der Benutzer die Arbeitsabschnitte 34a-c durch Krafteinleitung in das Führungselement 25 bewegen, um die Stellung der Arbeitsabschnitte 34a-c anzupassen. Dazu kann der Benutzer durch Drehen des Führungselements 25 um dessen eigene Achse die Arbeitsabschnitte 34a-c um diese Achse rotieren. Durch betätigen des Führungselements 25 kann der Benutzer die Arbeitsabschnitte 34a-c vorzugsweise um eine Achse quer, insbesondere senkrecht, zur Längserstreckungsrichtung des Führungselements 25 schwenken, in der sich das Führungselement 25 außerhalb des Arbeitskanals 20 erstreckt. Die Anpassung der Position und/oder Orientierung der Arbeitsabschnitte 34a-c relativ zu dem distalen Ende 14 des Schaftes 12 mittels des Führungselements 25, die Behandlung und/oder die Untersuchung in dem Bereich 18 kann mittels der Vorrichtung 10 vorzugsweise unter visueller Kontrolle des Benutzers erfolgen, der den Bereich 18 von außerhalb des Körpers des Patienten durch das Mittel zur Bildübertragung einsehen kann.

Die erfindungsgemäße Vorrichtung 10 umfasst einen Endoskopschaft 12, durch den sich ein an einer Austrittsseite 16 des Schaftes 12 mündender Arbeitskanal 20 erstreckt. Durch den Arbeitskanal 20 erstreckt sich ein Führungselement 25, das in dem Arbeitskanal 20 vorzugsweise längsbeweglich entlang des Arbeitskanals 20 geführt ist. Die Vorrichtung 10 ist derart eingerichtet, dass der distale Endabschnitt 32a-c eines sich längs neben dem Schaft 12 erstreckenden Instruments 30a-c längs des Schaftes 12 über die Austrittsseite 16 hinaus bewegt werden kann, um den Arbeitsabschnitt 34, 34a-c des Instruments 30, 30a-c weg von der Austrittsseite 16 zu bewegen, um den distalen Endabschnitt der Vorrichtung 10 zu verlängern. Das Instrument 30, 30a-c ist mit dem Führungselement 25 gekoppelt, um den Arbeitsabschnitt 34, 34a-c des Instruments 30, 30a-30c in eine Richtung quer zur Austrittsseite 16, beispielsweise senkrecht zur Austrittsseite 16, von der Austrittsseite 16 weg in einen Bereich 18 gegenüber - dies schließt auch schräg gegenüber ein - der Austrittsseite 16 zu führen, um in dem Bereich 18 mit dem Instrument 30, 30a-c zu arbeiten. Auf Grund des in dem Arbeitskanal 20 gehaltenen und/oder geführten Führungselements 25 wird das Instrument 30, 30a-30c gegenüber der Austrittsseite 16 vermittelt über das Führungselement 25 von dem Schaft 12 gehalten und/oder mittels des Arbeitskanals 20 geführt. Die Vorrichtung 10 ist vorzugsweise derart eingerichtet, dass die Arbeitsabschnitte 34, 34a-d der Instrumente 30, 30a-c bei der Bewegung der Arbeitsabschnitte 34, 34a-c weg von der Austrittsseite 16 und/oder zurück zu dem Schaft 12 entlang der Längserstreckungsrichtung des distalen Endabschnitts 20a des Arbeitskanals 20 geführt verschoben und/oder gezogen werden. Dadurch werden eine besonders stabile Anordnung des neben dem Schaft 12 über den Schaft 12 hinausstehenden Instruments 30, 30a-30c und/oder eine besonders stabile Führung des Instruments 30, 30a-c über den Schaft 12 hinaus erhalten, die eine genaue und zügige Positionierung des Arbeitsabschnitts 34, 34a-c des Instruments 30, 30a-c ermöglicht.

### Bezugszeichenliste:

| | |
|---|---|
| 10 | Vorrichtung |
| 11 | Endoskop |
| 12 | Schaft |
| 13 | distale Richtung |
| 14 | distales Ende |
| 15 | proximale Richtung |
| 16 | Austrittsseite, Stirnseite |
| 17 | Beleuchtungseinheit |
| 18 | Bereich |
| 19 | Eingang des Mittels zur Bildübertragung |
| 20 | Arbeitskanal |
| 20a | Endabschnitt des Arbeitskanals |
| 21 | Öffnung |
| 25 | Führungselement |
| 26 | Längsrichtung |
| 30 | Instrument |
| 30a | erstes Instrument |
| 30b | zweites Instrument |
| 30c | drittes Instrument |
| 32 | distales Ende |
| 32a-c | distale Endabschnitte |
| 34 | Arbeitsabschnitt |
| 34a-c | Arbeitsabschnitte |
| 36 | Funktionselement |
| 36a-c | Funktionselemente |
| 40 | Führungsmanschette, erster Führungshalter |
| 40i, 40ii, 40iii | Aufnahmen |
| 41 | Außenseite, Längsseite |
| 42 | Längserstreckungsrichtung |
| 43 | zweiter Führungshalter |
| 44 | distales Ende der Vorrichtung |
| 45 | Eingriffselement |
| 45a | Formschlussabschnitt |
| | |
| 48 | Abschnitt |
| 50 | Führungsrichtung |
| 52 | Längserstreckungsrichtung |
| 55 | Doppelpfeil |
| 56 | Doppelpfeil |
| 57 | distales Ende des Führungselements |
| 60 | Aufnahme |
| 61 | Aufnahme |
| 65 | Halteelement |
| 65a | Formschlussabschnitt |
| 70 | Eingriffsabschnitt |
| 70a, 70b | Eingriffsabschnitte |
| 71a, 71b | Doppelpfeile |
| 72a,b | Doppelpfeile |
| 75 | Schnappelement, Schnappzunge |
| 76 | Formschlussflächen |
| 77 | Körper |
| 80 | Handgriff |
| 81 | proximales Ende |
| 82 | Führungshalter |
| 83 | Bedienteil |
| 84a, 84b | Abwinkelungsräder |
| 85 | Bedienelemente |
| 86 | Eingang |
| 87 | Führungselementgriff |
| 88 | Doppelpfeil |
| 89 | Doppelpfeil |
| 90 | Öffnung |
| 91 | Instrument |

## Patentansprüche

1. Vorrichtung (10) mit einem Endoskop (11), welches einen Schaft (12) umfasst, wobei der Schaft (12) des Endoskops (11) einen Arbeitskanal (20) umgrenzt, der an einer Seite (16) des Schaftes (12) mündet,
wobei die Vorrichtung (10) ein Instrument (30, 30a, 30b, 30c) aufweist, das neben dem Schaft (12) verlaufend angeordnet ist, wobei die Vorrichtung (10) derart eingerichtet ist, dass ein Endabschnitt (32, 32a, 32b, 32c) des Instruments (30, 30a, 30b, 30c), der einen Arbeitsabschnitt (34, 34a, 34b, 34c) des Instruments (30, 30a, 30b, 30c) enthält, entlang des Schaftes (12) über die Seite (16) des Schaftes (12) hinaus bewegbar ist, um den Arbeitsabschnitt (34, 34a, 34b, 34c) des Instruments (30, 30a, 30b, 30c) in einen Bereich (18) gegenüber der Seite (16) des Schaftes (12) zu bewegen, um mit dem Arbeitsabschnitt (34, 34a, 34b, 34c) in dem Bereich (18) zu arbeiten,
wobei das Instrument (30, 30a, 30b, 30c) mit einem Führungselement (25) in Eingriff steht, um den Arbeitsabschnitt (34, 34a, 34b, 34c) des Instruments (30, 30a, 30b, 30c) bei der Bewegung des Endabschnitts (32, 32a, 32b, 32c) über die Seite (16) des Schaftes (12) hinaus in den Bereich (18) zu führen,
wobei sich das Führungselement (25) zur Führung des Arbeitsabschnitts (34, 34a, 34b, 34c) des Instruments (30, 30a, 30b, 30c) bei der Bewegung des distalen Endabschnitts (32, 32a, 32b, 32c) über die Seite (16) des Schaftes (12) hinaus durch den Arbeitskanal (20) erstreckt, wobei das Instrument (30, 30a, 30b, 30c) ein chirurgisches Instrument (30, 30a, 30b, 30c) ist, welches derart mit dem Führungselement (25) bewegungsgekoppelt ist, so dass ein Vorschieben des Führungselements (25) in dem Arbeitskanal (20) in Längserstreckungsrichtung des Arbeitskanals (20) in Richtung zu der Seite (16) in eine Bewegung des Arbeitsabschnitts (34, 34a, 34b, 34c) des Instruments (30, 30a, 30b, 30c) von der Seite (16) weg umgesetzt wird, und
wobei der Arbeitsabschnitt (30, 30a, 30b, 30c) um eine entlang der Längserstreckungrichtung des Führungselements (25) durch das Führungselement (25) verlaufende Achse relative zu dem Schaft (12) rotiert werden kann und um eine quer zu dem Führungselement (25) verlaufende Achse geschwenkt werden kann, um die Orientierung der gegenüber der Stirnseite (16) angeordneten Arbeitsabschnitte (34a, 34b, 34c) zu verändern.

2. Vorrichtung (10) nach einem der vorhergehenden Ansprüche, wobei an der Längsseite (41) des Schaftes (12) eine Führung zur Führung des Instruments (30, 30a, 30b, 30c) entlang des Schaftes (12) angeordnet ist.

3. Vorrichtung (10) nach einem der vorhergehenden Ansprüche, wobei das Führungselement (25) zum Eingriff mit wenigstens zwei Instrumenten (30a, 30b, 30c) eingerichtet ist.

4. Vorrichtung (10) nach einem der vorhergehenden Ansprüche, wobei an dem Führungselement (25) ein Eingriffselement (45) befestigt ist, das mit dem wenigstens einem Instrument (30a, 30b) in Eingriff steht.

5. Vorrichtung (10) nach Anspruch 4, wobei das Instrument (30, 30a, 30b, 30c) derart mit dem Eingriffselement (45) und/oder einem Eingriffsabschnitt (70, 70a, 70b) in Eingriff steht, dass das Instrument (30, 30a, 30b, 30c) relativ zu dem Eingriffselement (45) und/oder dem Eingriffsabschnitt (70, 70a, 70c) bewegbar in dem Eingriffselement (45) und/oder dem Eingriffsabschnitt (70, 70a, 70b) verschiebbar geführt ist.

6. Vorrichtung (10) nach Anspruch 5, wobei das Eingriffselement (45) und/oder der Eingriffsabschnitt (70, 70a, 70b) eine zu der äußeren Form des distalen Endes (14) des Schaftes (12) passende Form aufweist und/oder eine zu einem an dem distalen Ende (14) des Schaftes (12) angeordneten Halteelement (65) passende Form aufweist, so dass das Eingriffselement (45) und/oder der Eingriffsabschnitt (70, 70a, 70b) eine festgelegte Orientierung und/oder eine festgelegte Position relativ zu dem distalen Ende (14) des Schaftes (12) und/oder relativ zu dem Halteelement (65) aufweist, wenn die passende Form mit der äußeren Form in Eingriff steht.

7. Vorrichtung (10) nach einem der vorhergehenden Ansprüche, wobei der Eingriff zwischen dem Führungselement (25) und dem Instrument (30, 30a, 30b, 30c) zerstörungsfrei lösbar ist und wobei, nach dem Lösen des Eingriffs, ein zerstörungsfrei lösbarer Eingriff zu einem anderen Instrument (30, 30a, 30b, 30c) herstellbar ist.

8. Vorrichtung (10) nach einem der vorhergehenden Ansprüche, wobei ein an dem distalen Ende (14) des Schaftes (12) befestigter Führungshalter (40) Aufnahmen (40i, 40ii, 40iii) aufweist, in denen je ein Instrument (30, 30a, 30b, 30c) verschiebbar geführt ist.

9. Vorrichtung (10) nach einem der vorhergehenden Ansprüche, wobei das Führungselement (25) zur Aufnahme wenigstens zweier Instrumente (30a, 30b), die neben dem Schaft (12) verlaufend angeordnet sind, derart eingerichtet ist, dass der Arbeitsabschnitt (34a, 34b) wenigstens eines der Instrumente (30a, 30b) relativ zu dem Arbeitsabschnitt (34a, 34b) eines anderen der Instrumente (30a, 30b) mittels des Führungselements geführt verschiebbar ist und/oder wobei der Arbeitsabschnitt (34a, 34b) wenigstens eines der Instrumente (30a, 30b) relativ zu dem Arbeitsabschnitt (34a, 34b) des anderen Instruments (30a, 30b) mittels des Führungselements (25) drehbar und/oder schwenkbar ist.

## Claims

1. Device (10) with an endoscope (11) comprising a shaft (12), wherein the shaft (12) of the endoscope (11) encloses a working channel (20) that opens on one side (16) of the shaft (12),
wherein the device (10) comprises an instrument (30, 30a, 30b, 30c) that is arranged so as to extend next to the shaft (12), wherein the device (10) is configured in such a manner that an end section (32, 32a, 32b, 32c) of the instrument (30, 30a, 30b, 30c) containing a working section (34, 34a, 34b, 34c) of the instrument (30, 30a, 30b, 30c) can be moved along the shaft (12) beyond the side (16) of the shaft (12) in order to move the working section (34, 34a, 34b, 34c) of the instrument (30, 30a, 30b, 30c) into a region (18) opposite the side (16) of the shaft (12) so as to be able to work with the working section (34, 34a, 34b, 34c) in the region (18),
wherein the instrument (30, 30a, 30b, 30c) is in engagement with a guiding element (25) in order to guide the working section (34, 34a, 34b, 34c) of the instrument (30, 30a, 30b, 30c), when the end section (32, 32a, 32b, 32c) is being moved, beyond the side (16) of the shaft (12), into the region (18),
wherein the guiding element (25) for guiding the working section (34, 34a, 34b, 34c) of the instrument (30, 30a, 30b, 30c) extends through the working channel (20), when the distal end section (32, 32a, 32b, 32c) is being moved beyond the side (16) of the shaft (12),
wherein the instrument (30, 30a, 30b, 30c) is a surgical instrument (30, 30a, 30b, 30c) which is movably coupled with the guiding element (25) such that forward movement of the guiding element (25) in the working channel (20) in the longitudinal extension direction of the working channel (20) towards the side (16) is converted into movement of the working section (34, 34a, 34b, 34c) of the instrument (30, 30a, 30b, 30c) away from the side (16),
and wherein the working section (30, 30a, 30b, 30c) can be rotated relative to the shaft (12) about an axis extending along the longitudinal extension direction of the guiding element (25) through the guiding element (25) and can be pivoted about an axis extending transversely to the guiding element (25) in order to change the orientation of the working sections (34a, 34b, 34c) arranged opposite the end face (16).

2. Device (10) according to one of the previous claims,
wherein a guide for guiding the instrument (30, 30a, 30b, 30c) along the shaft (12) is provided on the longitudinal side (41) of the shaft (12).

3. Device (10) according to one of the previous claims,
wherein the guiding element (25) is configured for engagement with at least two instruments (30a, 30b, 30c).

4. Device (10) according to one of the previous claims,
wherein an engagement element (45) is fastened to the guiding element (25), said engagement element being in engagement with the at least one instrument (30a, 30b).

5. Device (10) according to claim 4, wherein the instrument (30, 30a, 30b, 30c) is in engagement with the engagement element (45) and/or the engagement section (70, 70a, 70b) in such a manner that the instrument (30, 30a, 30b, 30c) can be moved relative to the engagement element (45) and/or the engagement section (70, 70a, 70c) in the engagement element(45), and/or can be guided in a sliding manner in the engagement section (70, 70a, 70b).

6. Device (10) according to claim 5, wherein the engagement element (45) and/or the engagement section (70, 70a, 70b) have a form matching the outer form of the distal end (14) of the shaft (12), and/or a form matching a holding element (65) arranged on the distal end (14) of the shaft, so that the engagement element (45) and/or the engagement section (70, 70a, 70b) exhibit a fixed orientation and/or a fixed position relative to the distal end (14) of the shaft (12) and/or relative to the holding element (65) when the matching form is in engagement with the outer form.

7. Device (10) according to one of the previous claims,
wherein the engagement between the guiding element (25) and the instrument (30, 30a, 30b, 30c) can be released in a non-destructive manner and wherein, after the engagement is released, a non-destructively releasable engagement with another instrument (30, 30a, 30b, 30c) can be created.

8. Device (10) according to one of the previous claims,
wherein a guide holder (40) fastened to the distal end (14) of the shaft (12) has receptacles (40i, 40ii, 40iii) in which one instrument (30, 30a, 30b, 30c), respectively, is guided in a slidable manner.

9. Device (10) according to one of the previous claims,
wherein the guiding element (25) for the accommodation of at least two instruments (30a, 30b) arranged extending next to the shaft (12) is configured in such a manner that the working section (34a, 34b) of at least one of the instruments (30a, 30b) can be moved relative to the working section (34a, 34b) of another instrument (30a, 30b) guided by means of the guiding element, and/or wherein the working section (34a, 34b) of at least one of the instruments (30a, 30b) can be rotated and/or pivoted relative to the working section (34a, 34b) of the other instrument (30a, 30b) by means of the guiding element (25).

## Revendications

1. Dispositif (10) doté d'un endoscope (11) qui comporte une tige (12), la tige (12) de l'endoscope (11) délimitant un canal de travail (20) qui débouche sur une face (16) de la tige (12),
dans lequel le dispositif (10) présente un instrument (30, 30a, 30b, 30c) qui est placé de manière à s'étendre à côté de la tige (12), le dispositif (10) étant conçu de façon à ce qu'une partie d'extrémité (32, 32a, 32b, 32c) de l'instrument (30, 30a, 30b, 30c), qui comporte une partie de travail (34, 34a, 34b, 34c) de l'instrument (30, 30a, 30b, 30c), puisse être déplacée le long de la tige (12), au-delà de la face (16) de la tige (12), en vue de déplacer la partie de travail (34, 34a, 34b, 34c) de l'instrument (30, 30a, 30b, 30c) pour l'amener dans une région (18) située en vis-à-vis de la face (16) de la tige (12), afin de coopérer avec la partie de travail (34, 34a, 34b, 34c) dans la région (18),
dans lequel l'instrument (30, 30a, 30b, 30c) est en prise avec un élément de guidage (25), afin de guider la partie de travail (34, 34a, 34b, 34c) de l'instrument (30, 30a, 30b, 30c) au-delà de la face (16) de la tige (12), pour l'amener dans la région (18), lors du déplacement de la partie d'extrémité (32, 32a, 32b, 32c),
dans lequel l'élément de guidage (25), aux fins de guider la partie de travail (34, 34a, 34b, 34c) de l'instrument (30, 30a, 30b, 30c), lors du mouvement de la partie d'extrémité (32, 32a, 32b, 32c) distale au-delà de la face (16) de la tige (12), s'étend dans le canal de travail (20),
dans lequel l'instrument (30, 30a, 30b, 30c) est un instrument chirurgical (30, 30a, 30b, 30c) qui est couplé en termes de mouvement à l'élément de guidage (25) de manière à ce qu'un coulissement de l'élément de guidage (25) vers l'avant dans le canal de travail (20), dans le sens longitudinal du canal de travail (20), en direction de la face (16), soit transformé en un mouvement de la partie de travail (34, 34a, 34b, 34c) de l'instrument (30, 30a, 30b, 30c) dans le sens opposé à la face (16), et
dans lequel la partie de travail (30, 30a, 30b, 30c) peut être mise en rotation par rapport à la tige (12), autour d'un axe s'étendant à travers l'élément de guidage (25), le long de la direction longitudinale de l'élément de guidage (25), et peut être pivotée autour d'un axe s'étendant transversalement à l'élément de guidage (25), aux fins de modifier l'orientation des parties de travail (34a, 34b, 34c) disposées en vis-à-vis de la face frontale (16).

2. Dispositif (10) selon l'une des revendications précédentes, dans lequel un guide, destiné au guidage de l'instrument (30, 30a, 30b, 30c) le long de la tige (12), est disposé sur le côté longitudinal (41) de la tige (12).

3. Dispositif (10) selon l'une des revendications précédentes, dans lequel l'élément de guidage (25) est conçu pour entrer en prise avec au moins deux instruments (30a, 30b, 30c).

4. Dispositif (10) selon l'une des revendications précédentes, dans lequel est fixé à l'élément de guidage (25), un élément d'engagement (45) qui est en prise avec l'instrument (30a, 30b), au nombre d'au moins un.

5. Dispositif (10) selon la revendication 4, dans lequel l'instrument (30, 30a, 30b, 30c) est en prise avec l'élément d'engagement (45) et/ou une partie d'engagement (70, 70a, 70b), de manière à ce que l'instrument (30, 30a, 30b, 30c) soit guidé avec possibilité de coulissement dans l'élément d'engagement (45) et/ou la partie d'engagement (70, 70a, 70b), de façon à pouvoir être déplacé par rapport à l'élément d'engagement (45) et/ou la partie d'engagement (70, 70a, 70b).

6. Dispositif (10) selon la revendication 5, dans lequel l'élément d'engagement (45) et/ou la partie d'engagement (70, 70a, 70b) présente une forme qui est adaptée à la forme extérieure de l'extrémité distale (14) de la tige (12), et/ou présente une forme qui est adaptée à un élément de support (65) disposé à l'extrémité distale (14) de la tige (12), de sorte que l'élément d'engagement (45) et/ou la partie d'engagement (70, 70a, 70b) présente une orientation définie et/ou une position définie par rapport à l'extrémité distale (14) de la tige (12) et/ou par rapport à l'élément de support (65), lorsque la forme adaptée est en prise avec la forme extérieure.

7. Dispositif (10) selon l'une des revendications précédentes, dans lequel la prise entre l'élément de guidage (25) et l'instrument (30, 30a, 30b, 30c) peut être libérée sans destruction, et dans lequel, après la libération de la prise, il est possible d'établir une prise libérable sans destruction avec un autre instrument (30, 30a, 30b, 30c).

8. Dispositif (10) selon l'une des revendications précédentes, dans lequel un support de guidage (40) fixé à l'extrémité distale (14) de la tige (12) présente des logements (40i, 40ii, 40iii) dans lesquels respectivement un instrument (30, 30a, 30b, 30c) est guidé de manière coulissante.

9. Dispositif (10) selon l'une des revendications précédentes, dans lequel l'élément de guidage (25) est conçu pour accueillir au moins deux instruments (30a, 30b), qui sont disposés de manière à s'étendre à côté de la tige (12), de telle sorte que la partie de travail (34a, 34b) d'au moins un des instruments (30a, 30b) puisse être coulissée par rapport à la partie de travail (34a, 34b) d'un des autres instruments (30a, 30b), en étant guidée à l'aide de l'élément de guidage, et/ou dans lequel la partie de travail (34a, 34b) d'au moins un des instruments (30a, 30b) peut être mise en rotation et/ou être pivotée par rapport à la partie de travail (34a, 34b) de l'autre instrument (30a, 30b), à l'aide de l'élément de guidage (25).
